# EUROPEAN PATENT APPLICATION

(11) **EP 3 549 531 A1**
(43) Date of publication of application: **09.10.2019**
(21) Application number: 17875842.1
(22) Date of filing: 13.10.2017
(51) Int. Cl.: A61B 10/02

(54) **ROTARY CUTTING TOOL AND ROTARY CUTTING OPERATING ASSEMBLY**

(30) Priority: 30.11.2016 CN 201611094251; 30.11.2016 CN 201611085595
(71) Applicant: Chongqing Xishan Science & Technology Co.,Ltd., Chongqing 401121 (CN)
(72) Inventor: GUO, Yijun, Chongqing 401121 (CN); ZHANG, Jinbin, Chongqing 401121 (CN); ZHANG, Xinyun, Chongqing 401121 (CN); LI, Hongyuan, Chongqing 401121 (CN); YANG, Yongbo, Chongqing 401121 (CN)
(74) Representative: Mohun, Stephen John
(86) International application number: PCT/CN2017/106068
(87) International publication number: WO 2018/099195

(57) **Abstract**

A rotary cutting tool (1) and a rotary cutting operating assembly, the cutting tool (1) at least comprising an outer cutter (3) provided with a sampling groove (5) at the front end, and an inner cutter (4) sleeved inside the outer cutter (3); the cutting tool (1) also comprises a rotating driven member (6) slidably engaged with the inner cutter (4) in the circumferential transmission axial direction and a translational driven member (7) fitted to the inner cutter (4) by means of a lead screw structure; a handle (2) comprises a handle body and a drive component mounted on the handle body, the drive component comprising a rotating active member (8) used for driving the rotation of the rotating driven member (6) and a translational active member (9) used for driving the translational driven member (7); the rotating driven member (6) drives the rotation of the inner cutter (4) and forms a rotating speed differential with the translational driven member (7), the rotating speed differential driving the inner cutter (4) to move axially and thereby implement sampling of the sampling groove (5); the present rotating cutting tool (1) is structurally simple, easy to assemble, easy to operate, has high sampling efficiency, ensures successful operation, and also ensures that interference does not easily occur between the rotating movement and the translational movement; the running of the operating components is smooth and stable, the rotary cutting operation is highly efficient, and safe operation is ensured.

## Description

### TECHNICAL FIELD

The present disclosure relates to a medical surgical biopsy sampling equipment, in particular to a rotary cutting tool. The present disclosure also relates to a biopsy sampling apparatus for medical surgery, in particular to a rotary cutting operation assembly.

### BACKGROUND

During an operation, a doctor usually adopts a rotary cutting tool to perform live sampling or removal of the lesion tissue. Nowadays, the operation components for rotary cutting operation includes a cutting tool and a handle. The cutting tool usually includes an inner blade and an outer blade sleeved on the inner blade. A sampling slot is provided at a front end of the outer blade in the radial direction. After puncturing the body, the tissue is sucked into the sampling slot under the condition of negative pressure, and the inner blade rotates and cut forward and the tissue is cut and received into the front end of the inner blade. Of course, the inner blade will move to the foremost end of the outer blade to close the sampling slot during puncturing, and the inner blade will move backward to open the sampling slot when the sampling slot is in a target position. The position to open the sampling slot and moving distance of the inner blade is determined according to the size of the sampling. Negative pressure will be applied during the moving, and a rotary cutting sampling is then performed. In the prior art, the rotary motion and the translational motion of the inner blade are driven by separate and independent driving mechanisms. The inner blade is driven to rotate by a motor, while it is driven to slide axially by a push rod, thereby realizing the rotary cutting by combining the rotary motion and translational motion. The push rod may be driven electrically or manually. Manual operation is cumbersome and inefficient, and the electric operation required a decelerator to realize a slow pushing of the inner blade, which makes the rotary cutting operation component have a complex structure and heavy in overall weight. Moreover, the driving mechanisms for rotary motion and the translational motion of the inner blade is prone to interference with each other, which causes the inner blade to be locked, thereby resulting in unstable operation of the rotary cutting operation component, which is easy to bring hazard to the operation.

### SUMMARY

Accordingly, an object of the present disclosure is to address the defects in the prior art and provide a rotary cutting tool which can simplify the structure, improve the stability of the inner blade operation, and ensure the safety of the operation.

The rotary cutting tool according to the present disclosure includes an outer blade having a sampling slot at the front end thereof; an inner blade sleeved in the outer blade; a rotary driving member circumferentially transmission engaged with the inner blade and axially slidably engaged with the inner blade; and a translational driving member engaged with the inner blade via a screw means, wherein the rotary driving member drives the inner blade to rotate and forms a rotation speed difference with the translational driving member, the inner blade is driven to move axially by the rotation speed difference to implement sampling by the sampling slot.

Further, the cutting tool includes a rotary sleeve fixedly sleeved on the inner blade, the translational driving member is a translational driving gear that is sleeved on the rotary sleeve in a threaded engagement manner with the rotary sleeve.

Further, the rotary driving member is a rotary driving sleeve that is sleeved on the rotary sleeve in a radial limiting manner, and the rotary driving sleeve is provided with a rotary transmission gear on an outer circumference thereof. An outer circumference of the rotary sleeve extends radially and outwardly to form an annular boss that is circumferentially transmission engaged with and axially slidably engaged with an inner circumference of the rotary driving sleeve via a spline-slot structure.

Further, a rear end surface of the rotary driving sleeve abuts against a front end surface of the translational driving gear to form an axial limiting to the translational driving gear, an inner circumference of the rear end of the rotary driving sleeve protrudes radially and inwardly to form a support ring that is sleeved on an external thread on a rear end of the rotary sleeve.

Further, the inner circumference of the rotary driving sleeve is axially provided with a guiding spline, an outer circumference of the annular boss is provided with a sliding slot matching the guiding spline, a plurality of guiding splines and a plurality of sliding slots are distributed in a circumferential direction.

Further, the guiding spline and the sliding slot have a rectangular cross-section.

Further, a side wall of a front end of the inner blade is provided with a vent hole, negative pressure is generated at a rear end of the inner blade, such that air passes through a radial gap between the inner and outer blades, the vent hole, and an inner cavity of the inner blade successively to form an air flow channel.

Further, a puncture head is included whose rear end is inserted axially rearwardly and fixed to a front end of the outer blade, an outer circumference of the rear end of the puncture head is provided with a step, a front end of the inner blade is provided with a rotary cutter head inclined outwardly in a radial direction from the rear to the front, and a rear end of the step forms a slope that matches the inclination of the rotary cutter head for axially limiting the rotary cutter head.

Further, the rear end of the puncture head protrudes rearward in the axial direction to form a blocking post, and a radial gap is formed between the blocking post and an inner circumference of the inner blade.

Further, the vent hole is a strip hole provided in a circumferential direction, the strip hole is a vent ring extending in the circumferential direction, or a plurality of the strip holes are uniformly distributed in the circumferential direction to form a vent ring, and at least one vent ring is disposed in an axial direction.

The rotary cutting tool is disclosed in the present disclosure. The inner blade is driven by the rotary driving member and the translational driving member to perform a rotary movement and a translational movement in a differential rotation manner. Therefore, the need for a reducer for deceleration output, which results in a complex and heavier overall rotary cutting operation assembly structure, is avoided. The rotary cutting operation assembly has a simple structure and is easy to assemble, it is easy to operate in use and has high efficiency in sampling, which can ensure a successful operation. Meanwhile, the rotary movement and translational movement are not easy to be interfere with each other. The operating assembly runs smoothly and stably, and rotary cutting efficiency in operation is high, which is conducive to ensure the safety of the operation.

Another object of the present disclosure is to address the defects in the prior art and provide a rotary cutting operation assembly which can simplify the structure thereof, reduce the weight, improve the stability of the inner blade operation, and ensure the safety of the operation.

A rotary cutting operation assembly includes a cutting tool and a handle fixedly mounted to the cutting tool, wherein the cutting tool includes an outer blade having a sampling slot at the front end thereof; an inner blade sleeved in the outer blade; a rotary driven member circumferentially transmission engaged with the inner blade and axially slidably engaged with the inner blade; a translational driven member engaged with the inner blade via a screw means; wherein the handle comprises a handle body and a driver component mounted to the handle body, the driver component comprises a rotary active member configured to drive a rotary driven member and a translational active member configured to drive a translational driven member; wherein the rotary driven member drives the inner blade to rotate and forms a rotation speed difference with the translational driven member, the inner blade is driven to move axially by the rotation speed difference to implement sampling by the sampling slot.

Further, the rotary active member and the translational active member are a rotary active gear and a translational active gear, respectively, both ends of an inner circumference of the translational active gear extend axially to form a shaft sleeve, and the rotary active gear is sleeved on an end of the shaft sleeve in a circumferential transmission manner.

Further, the cutting tool includes a rotary sleeve fixedly sleeved on the inner blade, the translational driven member is a translational driving gear that is sleeved on the rotary sleeve in a threaded engagement manner with the rotary sleeve.

Further, the rotary driven member is a rotary driving sleeve that is sleeved on the rotary sleeve in a radial limiting manner, and the rotary driving sleeve is provided with a rotary transmission gear on an outer circumference thereof. An outer circumference of the rotary sleeve extends radially and outwardly to form an annular boss that is circumferentially transmission engaged with and axially slidably engaged with an inner circumference of the rotary driving sleeve via a spline-slot structure.

Further, a rear end surface of the rotary driving sleeve abuts against a front end surface of the translational driving gear to form an axial limiting to the translational driving gear, an inner circumference of the rear end of the rotary driving sleeve protrudes radially and inwardly to form a support ring that is sleeved on an external thread on a rear end of the rotary sleeve.

Further, an outer circumference of the rotary sleeve extends radially and outwardly to form an annular boss that is circumferentially transmission engaged with and axially slidably engaged with an inner circumference of the rotary driving sleeve via a spline-slot structure.

Further, the inner circumference of the rotary driving sleeve is axially provided with a guiding spline, an outer circumference of the annular boss is provided with a sliding slot matching the guiding spline, a plurality of guiding splines and a plurality of sliding slots are distributed in a circumferential direction.

Further, the rotary active member and the rotary driven member implement a power transmission by means of reduction transmission, and the translational active member and the translational driven member implement a power transmission by means of acceleration transmission.

Further, the rotary driven member is driven by the rotary active member via a direct gear engagement, and the translational driven member is driven by the translational active member via a direct gear engagement.

Further, the handle is provided with a display unit configured to display an opening length of the sampling slot in real time.

The rotary cutting operation assembly is disclosed in the present disclosure. Through the driving cooperation between the rotary active member and the rotary driven member, as well as the driving cooperation between the translational active member and the translational driven member, the inner blade is driven by the rotary driven member and the translational driven member to perform a rotary movement and a translational movement in a differential rotation manner. Therefore, the need for a reducer for deceleration output, which results in a complex and heavier overall rotary cutting operation assembly structure, is avoided. The rotary cutting operation assembly has a simple structure and is easy to assemble, it is easy to operate in use and has high efficiency in sampling, which can ensure a successful operation. Meanwhile, the rotary movement and translational movement are not easy to be interfere with each other. The operating assembly runs smoothly and stably, and rotary cutting efficiency in operation is high, which is conducive to ensure the safety of the operation.

### BRIEF DESCRIPTION OF THE DRAWINGS

The present disclosure is further described below in conjunction with the accompanying figures and embodiments:
Fig. 1 is a schematic view of a rotary cutting tool according to the present disclosure;
Fig. 2 is an enlarged view of portion A in Fig. 1;
Fig. 3 is a schematic view of a handle of a rotary cutting tool according to the present disclosure;
Fig. 4 is a schematic view of the rotary cutting tool after installation according to the present disclosure;
Fig. 5 is an enlarged view of portion B in Fig. 4;
Fig. 6 is an enlarged view of portion C in Fig. 5;
Fig. 7 is a schematic view of a rotary driving member and a translational driving member of the rotary cutting tool according to the present disclosure;
Fig. 8 is a left side view of Fig. 7;
Fig. 9 is a schematic view of the rotary cutting operation assembly according to the present disclosure;
Fig. 10 is another schematic view of the rotary cutting operation assembly according to the present disclosure;
Fig. 11 is a schematic view of a driving mechanism of the rotary cutting operation assembly according to the present disclosure; and
Fig. 12 is a left side view of Fig. 11.

### DETAILED DESCRIPTION OF THE EMBODIMENTS

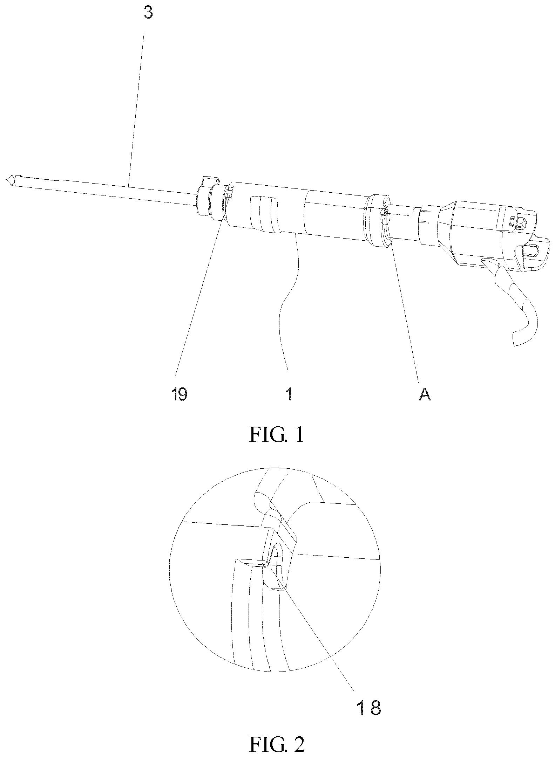

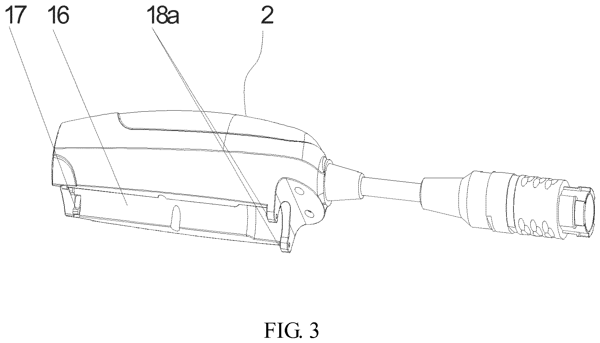

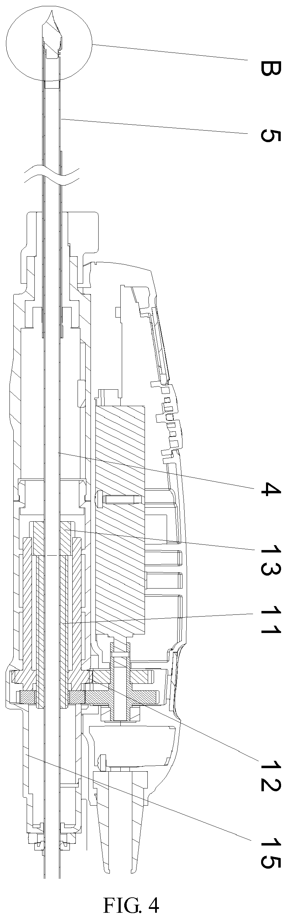

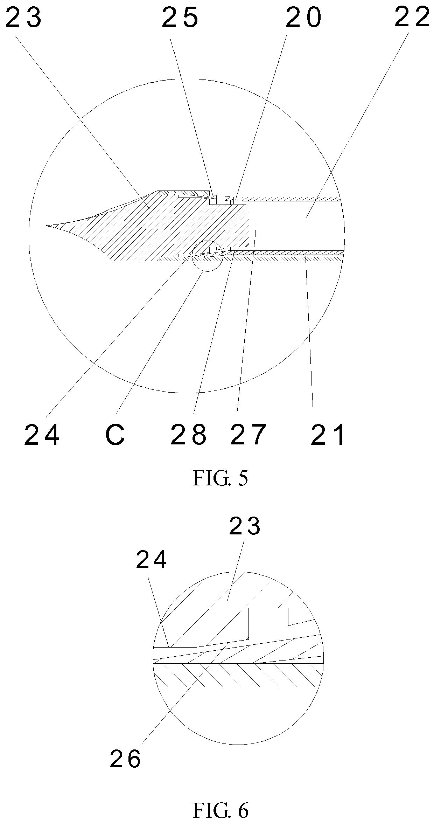

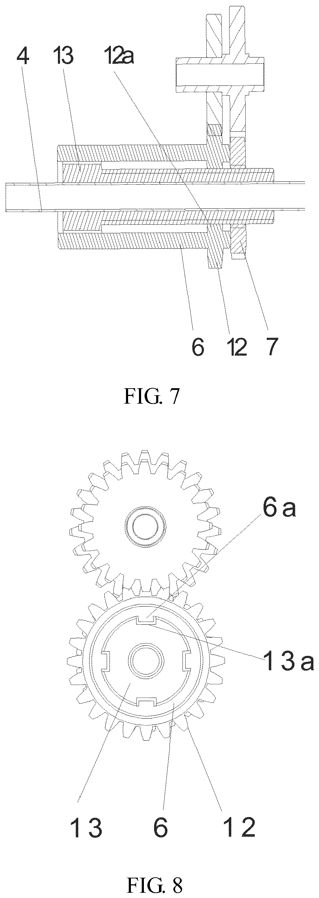

Referring to Fig. 1 to Fig. 8, Fig. 1 is a schematic view of a rotary cutting tool according to the present disclosure. Fig. 2 is an enlarged view of A in Fig. 1. Fig. 3 is a schematic view of a handle of a rotary cutting tool according to the present disclosure. Fig. 4 is a schematic view of the rotary cutting tool according to the present disclosure after installation. Fig. 5 is an enlarged view of B in Fig. 4. Fig. 6 is an enlarged view of C in Fig. 5. Fig. 7 is a schematic view of a rotary driving member and a translational driving member in a rotary cutting tool according to the present disclosure. Fig. 8 is a left side view of Fig. 7.

As illustrated in the figures, the rotary cutting tool 1 according to an embodiment includes: an outer blade 3 having a sampling slot 5 at a front end thereof, an inner blade 4 sleeved in the outer blade 3, and a shank 15 fixed to the outer blade 3. The rotary cutting tool 1 further includes a rotary driving member 6 circumferentially transmission engaged with the inner blade 4 and axially slidably engaged with the inner blade 4, and a translational driving member 7 which is engaged with the inner blade 4 via a screw means. The rotary driving member 6 drives the inner blade 4 to rotate which forms a rotation speed difference with the translational driving member 7, and the inner blade 4 is driven to move axially by the rotation speed difference to implement sampling by the sampling slot 5. A front end is the puncture end of the cutting tool and an opposite end is a rear end. A translational movement of the inner blade 4 means that the inner blade 4 reciprocates in an axial direction, which implements a rotary cutting movement in combination with a self-rotation of the inner blade 4. Of course, the different rotational speeds of the rotary driving member 6 and the translational driving member 7 can be achieved by configuring different transmission ratios or different rotational speeds driving of different motors. The rotary driving member 6 and the translational driving member 7 may have the same or different rotation directions. When they rotate in the same direction, an axial driving speed of the inner blade 4 is slow, while the axial driving speed of the inner blade 4 is faster when the directions are different. The inner blade 4 being driven by the rotary driving member 6 and translational driving member 7 in a differential rotation manner can simplify the structure of the rotary cutting tool and improve the stability of inner blade 4 in operation, thus ensuing safety of a surgery. The screw means is a conventional driving mechanism and that will not be described in greater details.

In this embodiment, the rotary cutting tool 1 further includes a rotary sleeve 11 fixedly sleeved on the inner blade 4. The translational driving member 7 is a translational driving gear 7 that is sleeved on the rotary sleeve 11 in a threaded engagement manner with the rotary sleeve 11. An outer circumference of the rotary sleeve 11 is provided with an external thread, and the translational driving gear 7 can be sleeved on a middle portion or a rear end of the rotary sleeve 11, preferably sleeved on the rear end of the rotary sleeve 11. The external thread disposed on the outer circumference of the rotary sleeve 11 is located in the middle and rear portion of the rotary sleeve 11 to ensure a compact structure. As shown in figures, the translational driving gear 7 is fixedly disposed along the axial direction of the shank 15. Of course, the translational driving member 7 can also be driven by a belt or a chain, both of which can achieve the purpose of the present disclosure. In the present embodiment, a direct gear transmission is preferred, which has a simple structure and is convenient to install. The rotary sleeve 11 is driven to rotate via the screw means, and the inner blade 4 is then driven to rotate and cut, thereby ensuring a strong and stable driving force.

In the present embodiment, the rotary driving member 6 is a rotary driving sleeve 6 that is sleeved on the rotary sleeve 11 in a radial limiting manner. A rotary transmission gear 12 is provided on an outer circumference of a rear end of the rotary driving sleeve 6. An outer circumference of the rotary sleeve 11 extends radially and outwardly to form an annular boss 13 that is circumferentially transmission engaged with and axially slidably engaged with an inner circumference of the rotary driving sleeve 6 via a spline-slot structure. Preferably, the annular boss 13 is located on a front end of the outer circumference of the rotary sleeve 11. Of course, the annular boss 13 supports the rotary driving sleeve 6 radially and outwardly. By configuring the rotary driving sleeve 6, it can be ensured that the external thread of the rotary sleeve 11 interferes with the rotary driving sleeve 6. At the same time, an inner circumference of the rotary driving sleeve 6 is axially provided with a guiding spline or a sliding slot, which can be engaged with a sliding slot or a guiding spline of the annular boss 13. Thus, the rotary sleeve 11 is adapted for sliding in the axial direction relative to the rotary driving sleeve 6.

In the present embodiment, a rear end surface of the rotary driving sleeve 6 abuts against a front end surface of the translational driving gear 7 to form an axial limiting to the translational driving gear 7. An inner circumference of the rotary driving sleeve 6 protrudes radially and inwardly to form a support ring 12a that is sleeved on the external thread of the rotary sleeve 11. Preferable, the support ring 12a is located on a rear end of the inner circumference of rotary driving sleeve 6, which is conducive to reduce the friction between the rotary driving sleeve 6 and the translational driving gear 7 and between the rotary driving sleeve 6 and the rotary sleeve 11. The support ring 12a can be aligned with the rotary transmission gear 12 in a radial direction, which is beneficial to ensure the stability of the rotary driving sleeve 6 in the radial direction and is advantageous for improving the stability of the rotation of the rotary driving sleeve 6.

In this embodiment, the inner circumference of the rotary driving sleeve 6 is provided with a guiding spline 6a in axial direction. An outer circumference of the annular boss 13 is provided with a sliding slot 13a matching the guiding spline 6a. The number of the guiding spline 16a and the sliding slot 13a are respectively plural and are distributed in a circumferential direction. In this embodiment, the number of the guiding spline 16a and sliding slot 13a are respectively four and are uniformly distributed in the circumferential direction, which can further improve the stability of the sliding of the rotary sleeve 11 and prevent locking.

In this embodiment, the cross-section of the spline-slot structure is rectangular. Thus the stability of the spline-slot structure is improved, the processing is convenient, and the matching precision is high.

In this embodiment, a side wall of a front end of the inner blade 4 is provided with a vent hole 20. Negative pressure is generated at the rear end of the inner blade 4, such that air passes through a radial gap 21 between the inner blade 4 and the outer blade 3, the vent hole 20, and an inner cavity 22 of the inner blade successively to form an air flow channel. The rear end of the inner blade 4 is connected to a suction apparatus. By configuring the vent hole 20, the inner blade 4 are prevented from being blocked at the front end, while liquid is prevented from entering the radial gap 21 between the inner blade 4 and the outer blade 3. The configuration of the radial gap 21 can ensure that the negative pressure at the front end of the inner cavity is moderate, which is favorable for the adsorption of the sample.

In this embodiment, the rotary cutting tool 1 further includes a puncture head 23 whose rear end is inserted axially rearwardly and fixed to the front end of the outer blade 3. The outer circumference of the rear end of the puncture head 23 is provided with a step 24. The front end of the inner blade 4 is provided with a rotary cutter head 25 inclined outwardly in a radial direction from the rear to the front. A rear end of the step 24 forms a slope 26 that matches the inclination of the rotary cutter head 25 for axially limiting the rotary cutter head 25. By providing the slope 26 at the rear end of the step 24 to axially limit the rotary cutter head 25, damage to the rotary cutter head 25 can be avoided, while ensuring that the sample can be completely cut.

In this embodiment, the rear end of the puncture head 23 protrudes rearward in the axial direction to form a blocking post 27. When the inner blade 4 moves to the most front end, a radial gap 28 is formed between the blocking post 27 and the inner circumference of the inner blade 4. The blocking post 27 is configured to form a structure such that the sample is gradually reduced from the rear to the front at the front end of the sampling slot 5, which facilitates the cutting of the sample at the slope 26 and ensures a smooth sampling.

In this embodiment, the vent hole 20 is a strip hole provided in a circumferential direction. The strip hole 20 is a vent ring extending in the circumferential direction. Alternatively, a plurality of strip holes are uniformly distributed in the circumferential direction to form the vent ring. At least one vent ring is disposed in an axial direction. In this embodiment, three strip holes are disposed in the circumferential direction and two vent holes are disposed in the axial direction. This structure is advantageous to ensure a large gas flow and smooth ventilation, while ensuring that the rotary cutter head 25 has high strength and is not easily damaged.

In use, the rotary cutting tool 1 is fixedly latched with the handle 2. A handle housing 14 of the handle 2 is provided with a mounting slot 16 in an axial direction. A rear end surface of the mounting slot 16 is provided with a pin 18a protruding forward in the axial direction. A front end of the mounting slot 16 is provided with a circlip 17 for fixing the shank 15. A rear end of the shank 15 is provided with an opening slot 18 matching with the pin 18a for radially fixing the shank 15. The outer circumference of the front end of the shank 15 is recessed radially inward to form a latching groove 19 for a radial claw of the circlip 17 to be embedded in. In the illustrated embodiment, the number of pins 18a and the opening slots 18 are both two, and the number of the radial claws and the latching grooves 19 of the circlip 17 are also two. During assembly, the shank 15 is firstly tilted and mounted backwards, so that the pin 18a is embedded into the opening slot 18. Then a front end of the shank 15 is moved upward, so that the front end of the shank 15 is embedded in the circlip 17, and the radial claw of the circlip 17 is embedded into the latching groove 19 to implement the latching and fixing. Conversely, during disassembling, the front end of the shank 15 is removed first and then the rear end thereof is removed, it is easy for quick assembly and disassembly, and the fixing is stable.

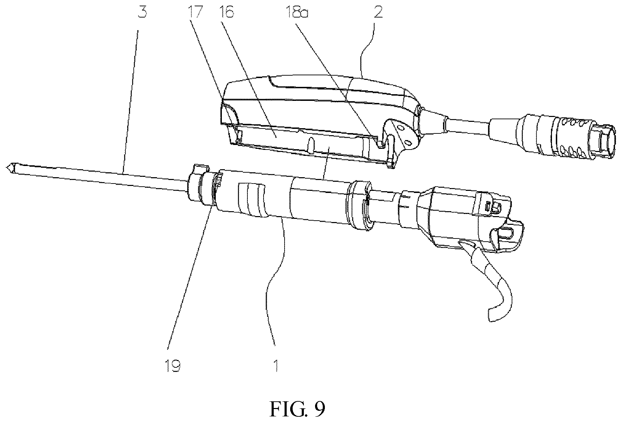

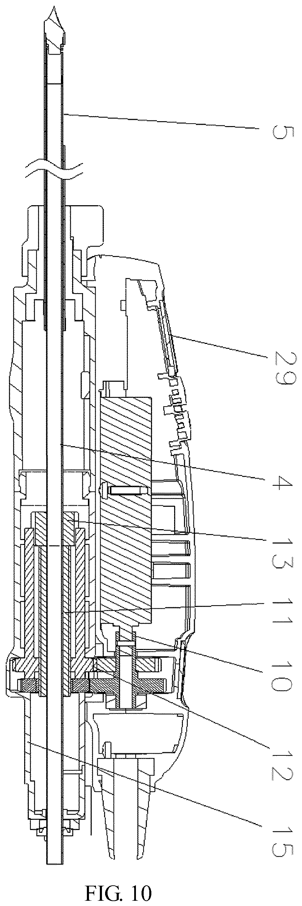

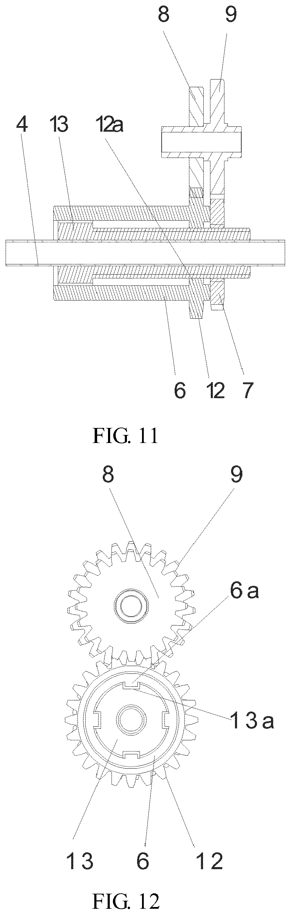

Referring to Fig. 9 to Fig. 12, Fig. 9 is a schematic view of a rotary cutting operation assembly according to the present disclosure. Fig. 10 is another schematic view of a rotary cutting operation assembly according to the present disclosure. Fig. 11 is a schematic view of a driving mechanism in a rotary cutting operation assembly according to the present disclosure. Fig. 12 is a left side view of Fig. 11.

As illustrated in figures, the rotary cutting operation assembly includes a cutting tool 1 and a handle 2 fixedly mounted to the cutting tool 1. The cutting tool 1 includes an outer blade 3 having a sampling slot 5 at a front end thereof, an inner blade 4 sleeved in the outer blade 3.

The cutting tool 1 above further includes a rotary driven member 6 circumferentially transmission engaged with the inner blade 4 and axially slidably engaged with the inner blade 4, and a translational driven member 7 which is engaged with the inner blade 4 via a screw means. The handle 2 includes a handle body and a driving component mounted on the handle body. The driving component includes a rotary active member 8 configured to drive the rotary driven member 6 and a translational active member 9 configured to drive the translational driven member 7. The rotary driven member 6 drives the inner blade 4 to rotate and forms a rotation speed difference with the translational driven member 7, and the inner blade 4 is driven to move axially by the rotation speed difference to implement sampling by the sampling slot 5. A front end is the puncture end of the cutting tool and an opposite end is a rear end. A translational movement of the inner blade 4 means that the inner blade 4 reciprocates in an axial direction, which implements a rotary cutting movement in combination with a self-rotation of the inner blade 4. Of course, the different rotational speeds of the rotary driven member 6 and the translational driven member 7 can be achieved by configuring different transmission ratios or different rotational speeds driving of different motors. The rotary driven member 6 and the translational driven member 7 may have the same or different rotation directions. When they rotate in the same direction, an axial driving speed of the inner blade 4 is slow, while the axial driving speed of the inner blade 4 is faster when the directions are different. The inner blade 4 being driven by the rotary driven member 6 and translational driven member 7 in a differential rotation manner can simplify the structure of the rotary cutting tool and improve the stability of inner blade 4 in operation, thus ensuing safety of a surgery. The screw means is a conventional driving mechanism and that will not be described in greater details.

In this embodiment, the rotary active member 8 and the rotary driven member 6 implement power transmission by means of reduction transmission. The rotary active member 8 transfers power to the rotary driven member 6 in a manner of reduction transmission, which is advantageous for increasing the rotational torque of the power input, ensuring that the rotary driven member 6 rotates smoothly, and avoiding the inner blade from being stopped by a large rotational resistance torque.

In this embodiment, the rotary active member 8 and the translational active member 9 are a rotary active gear 8 and a translational active gear 9, respectively. Both ends of the inner circumference of the translational active gear 9 extend axially to form a shaft sleeve 10. The rotary active gear 8 is sleeved on an end of the shaft sleeve 10 in a circumferential transmission manner. As shown in the figures, the front end of the shaft sleeve 10 is fixed to the shaft of the motor by a fixing pin. The rotary driving gear 8 is sleeved on the shaft sleeve 10 in a circumferential transmission manner via a spline that is located on a front circumferential end of the shaft sleeve 10, which ensures a compact structure and a stably driving.

In this embodiment, the translational active member 9 and the translational driven member 7 implement a power transmission by means of acceleration transmission. The translational active member 9 transfers power to the rotary driven member 6 in a manner of acceleration transmission, which is advantageous to be cooperate with the deceleration transmission mechanism of the rotary active member 8 and the rotary driven member 6 to realize a differential drive.

In this embodiment, the rotary driven member 6 is driven by the rotary active member 8 via a direct gear engagement. The translational driven member 6 is driven by the translational active member 9 via a direct gear engagement, which ensures precise transmission. Also, this configuration provides a high strength in transmission structure and ensures smooth power transmission.

In this embodiment, the rotary cutting tool 1 further includes a rotary sleeve 11 fixedly sleeved on the inner blade 4. The translational driven member 7 is a translational driving gear 7 that is sleeved on the rotary sleeve 11 in a threaded engagement manner with the rotary sleeve 11. An outer circumference of the rotary sleeve 11 is provided with an external thread, and the translational driving gear 7 can be sleeved on a middle portion or a rear end of the rotary sleeve 11, preferably sleeved on the rear end of the rotary sleeve 11. The external thread disposed on the outer circumference of the rotary sleeve 11 is located in the middle and rear portion of the rotary sleeve 11 to ensure a compact structure. As shown in figures, the translational driving gear 7 is fixedly disposed along the axial direction of the shank 15. Of course, the translational driven member 7 can also be driven by a belt or a chain, both of which can achieve the purpose of the present disclosure. In the present embodiment, a direct gear transmission is preferred, which has a simple structure and is convenient to install. The rotary sleeve 11 is driven to rotate via the screw means, and the inner blade 4 is then driven to rotate and cut, thereby ensuring a strong and stable driving force.

In the present embodiment, the rotary driven member 6 is a rotary driving sleeve 6 that is sleeved on the rotary sleeve 11 in a radial limiting manner. A rotary transmission gear 12 is provided on an outer circumference of a rear end of the rotary driving sleeve 6. An outer circumference of the rotary sleeve 11 extends radially and outwardly to form an annular boss 13 that is circumferentially transmission engaged with and axially slidably engaged with an inner circumference of the rotary driving sleeve 6 via a spline-slot structure. Preferably, the annular boss 13 is located on a front end of the outer circumference of the rotary sleeve 11. Of course, the annular boss 13 supports the rotary driving sleeve 6 radially and outwardly. By configuring the rotary driving sleeve 6, it can be ensured that the external thread of the rotary sleeve 11 interferes with the rotary driving sleeve 6. At the same time, an inner circumference of the rotary driving sleeve 6 is axially provided with a guiding spline or a sliding slot, which can be engaged with a sliding slot or a guiding spline of the annular boss 13. Thus, the rotary sleeve 11 is adapted for sliding in the axial direction relative to the rotary driving sleeve 6. As shown in figures, according to the present embodiment, the relationship between the number of teeth C of the rotary transmission gear 12, the number of teeth A of the rotary active gear 8, the number of teeth B of the translational active gear 9, and the number D of the teeth D of the translational driven gear 7 are: A<C, D<B, which can ensure a compact transmission structure and is advantageous to provide efficient rotary cutting drive force to the inner blade.

In the present embodiment, a rear end surface of the rotary driving sleeve 6 abuts against a front end surface of the translational driving gear 7 to form an axial limiting to the translational driving gear 7. An inner circumference of the rotary driving sleeve 6 protrudes radially and inwardly to form a support ring 12a that is sleeved on the external thread of the rotary sleeve 11. Preferable, the support ring 12a is located on a rear end of the inner circumference of rotary driving sleeve 6, which is conducive to reduce the friction between the rotary driving sleeve 6 and the translational driving gear 7 and between the rotary driving sleeve 6 and the rotary sleeve 11. The support ring 12a can be aligned with the rotary transmission gear 12 in a radial direction, which is beneficial to ensure the stability of the rotary driving sleeve 6 in the radial direction and is advantageous for improving the stability of the rotation of the rotary driving sleeve 6.

In this embodiment, the inner circumference of the rotary driving sleeve 6 is provided with a guiding spline 6a in axial direction. An outer circumference of the annular boss 13 is provided with a sliding slot 13a matching the guiding spline 6a. The number of the guiding spline 16a and the sliding slot 13a are respectively plural and are distributed in a circumferential direction. In this embodiment, the number of the guiding spline 16a and sliding slot 13a are respectively four and are uniformly distributed in the circumferential direction, which can further improve the stability of the sliding of the rotary sleeve 11 and prevent locking. In this embodiment, the cross-section of the spline-slot structure is rectangular. Thus the stability of the spline-slot structure is improved, the processing is convenient, and the matching precision is high.

In this embodiment, the handle 2 is provided with a display unit 29 for displaying an opening length of the sampling slot 5 in real time. The display unit 29 uses a row of LED chips, which can realize the purpose of visually observing the opening length of the sampling slot 5 by directly receiving the command of the host and lighting or extinguishing the LED according to the state of the synchronous display. Of course, a display screen mode can be adopted to realize the object of the present disclosure. The configuration above can provide straightforward and objective basis for the sampling, which will become an objective guide to the operator's sampling operation. Thus, the sampling process is precise in quantity and controllable, which will improve the efficient and reduce the patients' suffering. The method for monitoring the open length data of the sampling slot 5 and displaying in real time can be implemented by using the prior art, and details are not described herein.

In this embodiment, a side wall of a front end of the inner blade 4 is provided with a vent hole 20. Negative pressure is generated at the rear end of the inner blade 4, such that air passes through a radial gap 21 between the inner blade 4 and the outer blade 3, the vent hole 20, and an inner cavity 22 of the inner blade successively to form an air flow channel. The rear end of the inner blade 4 is connected to a suction apparatus. By configuring the vent hole 20, the inner blade 4 are prevented from being blocked at the front end, while liquid is prevented from entering the radial gap 21 between the inner blade 4 and the outer blade 3. The configuration of the radial gap 21 can ensure that the negative pressure at the front end of the inner cavity is moderate, which is favorable for the adsorption of the sample.

The vent hole 20 is a strip hole provided in a circumferential direction. Alternatively, a plurality of strip holes are uniformly distributed in the circumferential direction to form the vent ring. At least one vent ring is disposed in an axial direction. In this embodiment, three strip holes are disposed in the circumferential direction and two vent rings are disposed in the axial direction. This structure is advantageous to ensure a large gas flow and smooth ventilation, while ensuring that the rotary cutter head 25 has high strength and is not easily damaged.

the rotary cutting tool 1 further includes a puncture head 23 whose rear end is inserted axially rearwardly and fixed to the front end of the outer blade 3. The outer circumference of the rear end of the puncture head 23 is provided with a step 24. The front end of the inner blade 4 is provided with a rotary cutter head 25 inclined outwardly in a radial direction from the rear to the front. A rear end of the step 24 forms a slope 26 that matches the inclination of the rotary cutter head 25 for axially limiting the rotary cutter head 25. By providing the slope 26 at the rear end of the step 24 to axially limit the rotary cutter head 25, damage to the rotary cutter head 25 can be avoided, while ensuring that the sample can be completely cut.

In this embodiment, the rear end of the puncture head 23 protrudes rearward in the axial direction to form a blocking post 27. When the inner blade 4 moves to the most front end, a radial gap 28 is formed between the blocking post 27 and the inner circumference of the inner blade 4. The blocking post 27 is configured to form a structure such that the sample is gradually reduced from the rear to the front at the front end of the sampling slot 5, which facilitates the cutting of the sample at the slope 26 and ensures a smooth sampling.

In use, the rotary cutting tool 1 is fixedly latched with the handle 2. A handle housing 14 of the handle 2 is provided with a mounting slot 16 in an axial direction. A rear end surface of the mounting slot 16 is provided with a pin 18a protruding forward in the axial direction. A front end of the mounting slot 16 is provided with a circlip 17 for fixing the shank 15. A rear end of the shank 15 is provided with an opening slot 18 matching with the pin 18a for radially fixing the shank 15. The outer circumference of the front end of the shank 15 is recessed radially inward to form a latching groove 19 for a radial claw of the circlip 17 to be embedded in. In the illustrated embodiment, the number of pins 18a and the opening slots 18 are both two, and the number of the radial claws and the latching grooves 19 of the circlip 17 are also two. During assembly, the shank 15 is firstly tilted and mounted backwards, so that the pin 18a is embedded into the opening slot 18. Then a front end of the shank 15 is moved upward, so that the front end of the shank 15 is embedded in the circlip 17, and the radial claw of the circlip 17 is embedded into the latching groove 19 to implement the latching and fixing. Conversely, during disassembling, the front end of the shank 15 is removed first and then the rear end thereof is removed, it is easy for quick assembly and disassembly, and the fixing is stable.

The above embodiments are only used to illustrate the technical solutions of the present disclosure and are not intended to be limiting the scope of the present disclosure. Although the present disclosure has been described in details with reference to the preferred embodiments, those skilled in the art modifications or equivalents are intended to be included within the scope of the appended claims.

## Claims

1. A rotary cutting tool, comprising:
an outer blade having a sampling slot at the front end thereof;
an inner blade sleeved in the outer blade;
a rotary driving member circumferentially transmission engaged with the inner blade and axially slidably engaged with the inner blade; and
a translational driving member engaged with the inner blade via a screw means, wherein the rotary driving member drives the inner blade to rotate and forms a rotation speed difference with the translational driving member, the inner blade is driven to move axially by the rotation speed difference to implement sampling by the sampling slot; and
a rotary sleeve fixedly sleeved on the inner blade;
wherein the translational driving member is a translational driving gear that is sleeved on the rotary sleeve in a threaded engagement manner with the rotary sleeve, the rotary driving member is a rotary driving sleeve that is sleeved on the rotary sleeve in a radial limiting manner, and the rotary driving sleeve is provided with a rotary transmission gear on an outer circumference thereof.

2. The rotary cutting tool of claim 1, wherein a rear end surface of the rotary driving sleeve abuts against a front end surface of the translational driving gear to form an axial limiting to the translational driving gear, an inner circumference of the rear end of the rotary driving sleeve protrudes radially and inwardly to form a support ring that is sleeved on an external thread on a rear end of the rotary sleeve.

3. The rotary cutting tool of claim 1, wherein an outer circumference of the rotary sleeve extends radially and outwardly to form an annular boss that is circumferentially transmission engaged with and axially slidably engaged with an inner circumference of the rotary driving sleeve via a spline-slot structure.

4. The rotary cutting tool of claim 3, wherein the inner circumference of the rotary driving sleeve is axially provided with a guiding spline, an outer circumference of the annular boss is provided with a sliding slot matching the guiding spline, a plurality of guiding splines and a plurality of sliding slots are distributed in a circumferential direction.

5. The rotary cutting tool of claim 4, wherein the guiding spline and the sliding slot have a rectangular cross-section.

6. The rotary cutting tool of claim 1, wherein a side wall of a front end of the inner blade is provided with a vent hole, negative pressure is generated at a rear end of the inner blade, such that air passes through a radial gap between the inner and outer blades, the vent hole, and an inner cavity of the inner blade successively to form an air flow channel.

7. The rotary cutting tool of claim 1, further comprising a puncture head whose rear end is inserted axially rearwardly and fixed to a front end of the outer blade, an outer circumference of the rear end of the puncture head is provided with a step, a front end of the inner blade is provided with a rotary cutter head inclined outwardly in a radial direction from the rear to the front, and a rear end of the step forms a slope that matches the inclination of the rotary cutter head for axially limiting the rotary cutter head.

8. The rotary cutting tool of claim 7, wherein the rear end of the puncture head protrudes rearward in the axial direction to form a blocking post, and a radial gap is formed between the blocking post and an inner circumference of the inner blade.

9. The rotary cutting tool of claim 6, wherein the vent hole is a strip hole provided in a circumferential direction, the strip hole is a vent ring extending in the circumferential direction, or a plurality of the strip holes are uniformly distributed in the circumferential direction to form a vent ring, and at least one vent ring is disposed in an axial direction.

10. A rotary cutting operation assembly, comprising a cutting tool and a handle fixedly mounted to the cutting tool, wherein the cutting tool comprises:
an outer blade having a sampling slot at the front end thereof;
an inner blade sleeved in the outer blade;
a rotary driven member circumferentially transmission engaged with the inner blade and axially slidably engaged with the inner blade;
a translational driven member engaged with the inner blade via a screw means;
wherein the handle comprises a handle body and a driver component mounted to the handle body, the driver component comprises a rotary active member configured to drive a rotary driven member and a translational active member configured to drive a translational driven member;
wherein the rotary driven member drives the inner blade to rotate and forms a rotation speed difference with the translational driven member, the inner blade is driven to move axially by the rotation speed difference to implement sampling by the sampling slot;
the cutting tool comprises a rotary sleeve fixedly sleeved on the inner blade;
wherein the translational driven member is a translational driving gear that is sleeved on the rotary sleeve in a threaded engagement manner with the rotary sleeve, the rotary driven member is a rotary driving sleeve that is sleeved on the rotary sleeve in a radial limiting manner, and the rotary driving sleeve is provided with a rotary transmission gear on an outer circumference thereof.

11. The rotary cutting operation assembly of claim 10, wherein the rotary active member and the translational active member are a rotary active gear and a translational active gear, respectively, both ends of an inner circumference of the translational active gear extend axially to form a shaft sleeve, and the rotary active gear is sleeved on an end of the shaft sleeve in a circumferential transmission manner.

12. The rotary cutting operation assembly of claim 10, wherein the rotary active member and the rotary driven member implement a power transmission by means of reduction transmission, and the translational active member and the translational driven member implement a power transmission by means of acceleration transmission.

13. The rotary cutting operation assembly of claim 10, wherein the handle is provided with a display unit configured to display an opening length of the sampling slot in real time.

14. The rotary cutting operation assembly of claim 10, wherein the cutting tool is fixedly latched with the handle, a handle housing of the handle is provided with a mounting slot in an axial direction, a rear end surface of the mounting slot is provided with a pin protruding forward in the axial direction, a front end of the mounting slot is provided with a latching member for fixing a shank, a rear end of the shank is provided with an opening slot matching with the pin to fix the shank radially, an outer circumference of a front end of the shank is recessed radially inward to form a latching groove to embed the latching member.

15. The rotary cutting operation assembly of claim 14, wherein the latching member is a circlip provided with radial claw configured to be embedded into the latching groove.
